**Europäisches Patentamt**

**European Patent Office**

**Office européen des brevets**

(19)

(11) Veröffentlichungsnummer: **0 012 296 B1**

(12) **EUROPÄISCHE PATENTSCHRIFT**

(45) Veröffentlichungstag der Patentschrift:
02.06.82

(21) Anmeldenummer: **79104808.5**

(22) Anmeldetag: **01.12.79**

(51) Int. Cl.³: **C 07 C 87/30,** C 07 C 85/04, C 07 C 91/26, C 07 C 89/00

(54) **Verfahren zur Herstellung von quaternären Ammoniumhalogeniden in Pulver- oder Granulatform.**

(30) Priorität: **09.12.78 DE 2853241**

(43) Veröffentlichungstag der Anmeldung:
**25.06.80 Patentblatt 80/13**

(45) Bekanntmachung des Hinweises auf die Patenterteilung:
**02.06.82 Patentblatt 82/22**

(84) Benannte Vertragsstaaten:
**BE FR GB**

(56) Entgegenhaltungen:
**DD-A-59 792**
**DE-A-2 021 829**
**DE-A-2 406 965**
**DE-A1-2 625 945**
**DE-B2-2 244 297**

(73) Patentinhaber: **Henkel Kommanditgesellschaft auf Aktien, Postfach 1100 Henkelstrasse 67, D-4000 Düsseldorf 1 (DE)**

(72) Erfinder: **Giede, Wolfgang, Am Eichelkamp 6, D-4010 Hilden (DE)**
Erfinder: **Rutzen, Horst, Dr., Falkenweg 12, D-4018 Langenfeld (DE)**

## Verfahren zur Herstellung von quaternären Ammoniumhalogeniden in Pulver- oder Granulatform

Quaternäre Ammoniumhalogenide werden nach bekannten Verfahren durch Umsetzung von tertiären Aminen mit Alkylhalogeniden hergestellt. Diese Reaktion wird in der Regel in polaren Lösungsmitteln, vorzugsweise in Wasser, Äthanol, Isopropanol oder Gemischen dieser Lösungsmittel durchgeführt. Dabei müssen, insbesondere bei der Herstellung von quaternären Ammoniumhalogeniden, die einen längerkettigen Alkylrest aufweisen, beträchtliche Mengen an Lösungsmitteln eingesetzt werden, da diese Verbindungen nur begrenzt löslich sind und ihre konzentrierten Lösungen überdies die unangenehme Eigenschaft haben, in den Gelzustand überzugehen. So liegt die in der Praxis realisierbare Konzentrationsgrenze bei etwa 50 Gewichtsprozent; in vielen Fällen ist man jedoch gezwungen, bei Konzentrationen von 20 Gewichtsprozent und weniger zu arbeiten.

Bei der Verwendung von großen Mengen Lösungsmitteln wird die Kapazität der Reaktionsbehälter nicht in optimaler Weise genutzt. Werden die so hergestellten quaternären Ammoniumhalogenide durch Eindampfen von den Lösungsmitteln befreit, so bedeutet dies zusätzlichen Energieaufwand. Führt man diese Produkte ihrer Verwendung in der Form zu, in der sie anfallen, nämlich als Lösungen, so bedingt dies in den meisten Fällen einen zusätzlichen Aufwand an Transportmitteln.

Es wurde nun gefunden, daß man quaternäre Ammoniumhalogenide auf einfache Weise lösungsmittelfrei und in feinverteilter Form herstellen kann, wenn man nach dem nachstehend beschriebenen Verfahren arbeitet.

Es handelt sich dabei um ein Verfahren zur Herstellung von quaternären Ammoniumhalogeniden in Pulver- oder Granulatform durch Umsetzung von tertiären Aminen mit einem Überschuß an Alkylhalogeniden in Abwesenheit von Lösungsmitteln bei erhöhter Temperatur und erhöhtem Druck. Das neue Verfahren ist dadurch gekennzeichnet, daß man tertiäre Amine, die neben zwei Alkyl- oder Hydroxyalkylresten mit 1 bis 3 Kohlenstoffatomen einen Alkylrest oder $\beta$-Hydroxyalkylrest mit 8 bis 18 Kohlenstoffatomen enthalten, mit Methylchlorid, Methylbromid, Methyljodid, Ethylchlorid oder Ethylbromid zur Reaktion bringt und anschließend das unter Überdruck stehende heiße Reaktionsgemisch in einen unter geringerem Druck stehenden Behälter ausströmen läßt.

Als Beispiel für geeignete tertiäre Amine mit einem längerkettigen Alkylrest seien

Dimethyl-octylamin,
Dimethyl-decylamin,
Diethyl-decylamin,
Dimethyl-dodecylamin,
Di-n-propyl-dodecylamin,
Diethyl-tetradecylamin,
Dimethyl-hexadecylamin,

Di-n-propyl-hexadecylamin,
Dimethyl-octadecylamin,
Bis-(2-hydroxyethyl)-decylamin,
Bis-(2-hydroxypropyl)-dodecylamin,
Bis-(2-hydroxyethyl)-hexadecylamin,
Bis-(2-hydroxyethyl)-octadecylamin

genannt.

Als Ausgangsmaterial für die Herstellung von quaternären Ammoniumhalogeniden nach dem erfindungsgemäßen Verfahren eignen sich auch tertiäre Amine oder Gemische von tertiären Aminen mit einem längerkettigen $\beta$-Hydroxyalkylrest, wie sie nach bekannten Verfahren aus längerkettigen Epoxyalkanen oder Epoxyalkangemischen mit 8 bis 18 Kohlenstoffatomen und endständigen oder nichtendständigen Epoxygruppierungen durch Umsetzung mit Dialkylaminen wie Dimethylamin, Diethylamin und Di-n-propylamin sowie durch Umsetzung mit Dihydroxyalkylaminen wie Diethanolamin und Dipropanolamin erhalten werden können. Bei der Herstellung solcher tertiärer Amine werden vor allem Gemische von Epoxyalkanen mit unterschiedlicher Kettenlänge als Ausgangsstoffe eingesetzt. Bei den Epoxyalkanen mit nichtendständiger Epoxygruppierung werden überdies Gemische von Verbindungen bevorzugt, in denen die Epoxygruppierungen willkürlich über die an der Kohlenwasserstoffkette vorhandenen Innenpositionen verteilt sind. Als Beispiele für derartige tertiäre Amine seien angeführt:

Dimethyl-2-hydroxydecylamin,
Bis-(2-hydroxyethyl)-2-hydroxydecylamin,
Diethyl-2-hydroxydodecylamin,
Bis-(2-hydroxyethyl)-2-hydroxydodecylamin;
das Umsetzungsprodukt von Dimethylamin mit einem 1,2-Epoxyalkangemisch der Kettenlänge $C_{12-14}$,
das Umsetzungsprodukt von Diethylamin mit einem 1,2-Epoxyalkangemisch der Kettenlänge $C_{14-16}$,
das Umsetzungsprodukt von Di-n-propylamin mit einem 1,2-Epoxyalkangemisch der Kettenlänge $C_{16-18}$,
das Umsetzungsprodukt von Diethanolamin mit einem 1,2-Epoxyalkangemisch der Kettenlänge $C_{14-16}$,
das Umsetzungsprodukt von Dimethylamin mit einem Epoxyalkangemisch der Kettenlänge $C_{11-14}$ mit willkürlich verteilten, nichtendständigen Epoxygruppierungen,
das Umsetzungsprodukt von Diethylamin mit einem Epoxyalkangemisch der Kettenlänge $C_{14-16}$ mit willkürlich verteilten, nichtendständigen Epoxygruppierungen,
das Umsetzungsprodukt von Di-n-propylamin mit einem Epoxyalkangemisch der Kettenlänge $C_{15-18}$ mit willkürlich verteilten, nichtendständigen Epoxygruppierungen und das Umsetzungsprodukt von Diethanolamin mit einem Epoxyalkangemisch der Kettenlänge $C_{11-14}$ mit willkürlich verteilten, nichtendständigen Epoxygruppierungen.

Das als Quaternierungsmittel verwendete Alkylhalogenid wird in dem erfindungsgemäßen Verfahren immer im Überschuß eingesetzt. Vorzugsweise wird die Umsetzung bei einem Molverhältnis von tertiärem Amin zu Alkylhalogenid von 1 : 3 bis 1 : 8 durchgeführt.

Die Umsetzung zwischen tertiärem Amin und Alkylhalogenid wird unter den für diese Quaternierungsreaktionen üblichen Druck- und Temperaturbedingungen durchgeführt. In einer Vielzahl von Fällen ist der sich über dem Reaktionsgemisch einstellende autogene Druck für einen befriedigenden Ablauf der Reaktion ausreichend. Sofern dies erforderlich ist, kann der Druck durch Aufpressen von Inertgasen wie Stickstoff, Kohlendioxid, Methan, Ethan, Propan und Butan erhöht werden.

Als Reaktionsgefäße werden bei der Durchführung des erfindungsgemäßen Verfahrens normalerweise Druckreaktoren verwendet, die mit einer Heiz- und Kühleinrichtung, einem Rührwerk und einem geeigneten Ventil zum Ablassen des Reaktionsproduktes unter Druck ausgestattet sind.

Das aus der Quaternierungsreaktion resultierende Gemisch läßt man zweckmäßigerweise unmittelbar nach beendeter Reaktion ohne vorhergehende wesentliche Abkühlung und/oder Druckverminderung im Reaktor über das bereits erwähnte Ventil und gegebenenfalls durch eine geeignete Düse in einen Behälter ausströmen, der unter einem geringeren Druck als der Reaktor, vorzugsweise unter Normaldruck, steht. Dabei entspannt das im Reaktionsgemisch vorhandene überschüssige Alkylhalogenid augenblicklich, während das quaternäre Ammoniumhalogenid in feinverteilter, fester Form anfällt. Das entspannte Alkylhalogenid kann mit Hilfe eines Kompressors oder einer geeigneten Kühleranordnung kondensiert und einer erneuten Verwendung zugeführt werden. Selbstverständlich kann auch bereits vorgebildetes quaternäres Ammoniumhalogenid auf diese Weise in Pulver- oder Granulatform gebracht werden.

Das erfindungsgemäße Verfahren zur Herstellung von quaternären Ammoniumhalogeniden kann auch kontinuierlich durchgeführt werden. Dabei werden die Reaktionskomponenten in ein geheiztes Druckreaktionsrohr geeigneter Länge oder in eine geheizte Behälterkaskade eingepumpt. Am Ende der Reaktoreinheit befindet sich ein Entspannungsventil, das auf den vorgegebenen Reaktionsdruck eingestellt ist. Man läßt das Reaktionsgemisch in einen Tangentialabscheider ausströmen. Im unteren Teil des Abscheiders sammelt sich das quaternäre Ammoniumhalogenid, während das gasförmige Alkylhalogenid vom oberen Ende des Abscheiders abgesaugt und einem Kompressor oder einem Solekühler zugeführt wird.

Die nachstehenden Beispiele sollen den Gegenstand der Erfindung erläutern, ohne ihn hierauf einzuschränken.

### Beispiele

### Beispiel 1

In einem 1-l-Hubrührautoklaven mit Bodenventil wurden 225 g (1 Mol) $C_{8-18}$-Alkyl-dimethylamin (Dimethylkokosalkylamin; Aminzahl 249) und 303 g (6 Mol) Methylchlorid unter Rühren aufgeheizt bis bei 70°C die exotherme Reaktion einsetzte, wobei die Temperatur rasch auf 77°C anstieg, um dann wieder abzufallen. Anschließend wurde bei 70°C weitergerührt. 30 Minuten nachdem die Temperatur des Gemisches 70°C erreicht hatte, war die Aminzahl, wie in einer über das Bodenventil genommenen Probe festgestellt wurde, auf 1,1 abgefallen. Das bei 70°C unter einem Druck von 15 bar vorliegende $C_{8-18}$-Alkyl-trimethylammoniumchlorid wurde nun über das Bodenventil in eine 1-l-Pulverflasche abgelassen und wurde dabei als körniges weißes Produkt erhalten, das sich klar in Wasser löste.

### Beispiel 2

266 g (1 Mol) Dimethyl-hexadecylamin (Aminzahl 211) und 303 g (6 Mol) Methylchlorid wurden in einem 1-l-Autoklaven unter Rühren auf 70°C erwärmt, wobei der Druck auf 14 bar anstieg und eine positive Wärmetönung beobachtet wurde. Anschließend wurde bei 70°C weitergerührt. Nach 35 Minuten war die Reaktion abgeschlossen und die Aminzahl auf 3,2 abgefallen. Das bei 70°C unter einem Druck von 14 bar stehende Hexadecyltrimethylammoniumchlorid wurde direkt in eine Pulverflasche abgelassen und als rein weißes, feines Pulver erhalten. Das Produkt war in Wasser klar löslich und schäumte stark.

### Beispiel 3

239 g (0,9 Mol) Dimethyl-hexadecylamin (Aminzahl 211) und 271 g (4,2 Mol) Ethylbromid wurden in einem 1-l-Autoklaven unter Rühren auf 100°C erhitzt, wobei der Druck auf 13 bar anstieg. Das Reaktionsgemisch wurde 10 Stunden bei dieser Temperatur weitergerührt. Das entstandene Hexadecyl-trimethylammoniumbromid wurde dann ohne vorhergehende Abkühlung in eine Pulverflasche abgezogen und fiel als klar wasserlösliches Granulat an. Das Produkt war nicht hygroskopisch; nach der analytischen Untersuchung bestand es zu 95% aus dem quaternären Ammoniumhalogenid.

### Beispiel 4

Als Ausgangsmaterial diente ein Dimethyl-2-hydroxy-$C_{12-14}$-alkylamin (Aminzahl 218), das durch Umsetzung von Dimethylamin mit einem Gemisch von 1,2-Epoxyalkanen der Kettenlänge $C_{12-14}$ erhalten worden war. Auf 386 g (1,5 Mol) dieses tertiären Amins und 445 g (9 Mol) Methylchlorid in einem 1-l-Rührautoklaven wurde bis zu einem Druck von 10 bar Stickstoff aufgepreßt. Anschließend wurde der Autoklaveninhalt auf 80°C erhitzt, wobei der Druck auf 21 bar anstieg, und 5 Stunden auf dieser Temperatur gehalten. Das bei der Reaktion entstandene 2-Hydroxy-$C_{12-14}$-alkyl-trimethylammoniumchlorid wurde bei 75°C in eine Pulverflasche entspannt, wobei ein weißes, hygroskopisches Pulver mit der Aminzahl 2 erhalten wurde, das sich in Wasser klar löste.

### Beispiel 5

Als Ausgangsmaterial wurde ein Dimethyl-2-hydroxy-$C_{16-18}$-alkylamin (Aminzahl 180) verwendet, das durch Umsetzung Dimethylamin mit einem Gemisch von 1,2-Epoxyalkanen der Kettenlänge $C_{16-18}$ erhalten worden war. Auf 468 g (1,5 Mol) dieses tertiären Amins und 455 g (9 Mol) Methylchlorid in einem 1-l-Rührautoklaven wurde bis zu einem Druck von 10 bar Stickstoff aufgedrückt, bevor der Autoklaveninhalt auf 80°C erhitzt und 5 Stunden bei dieser Temperatur gehalten wurde. Das bei der Reaktion entstandene 2-Hydroxy-$C_{16-18}$-alkyl-trimethylammoniumchlorid, das bei 80°C unter einem Druck von 21 bar stand, wurde über das Bodenventil in eine Flasche entspannt und fiel als weißes hygroskopisches Pulver mit der Aminzahl 1,4 an.

### Beispiel 6

Als Ausgangsmaterial diente ein Dimethyl-$\beta$-hydroxy-$C_{11-14}$-alkylamin (Aminzahl 225), das aus einem Gemisch von $C_{11-14}$-Epoxyalkanen mit nichtendständigen Epoxygruppierungen in willkürlicher Verteilung durch Reaktion mit Dimethylamin erhalten worden war. 500 g (2 Mol) dieses Amins und 606 g (12 Mol) Methylchlorid wurden wie in Beispiel 5 unter einem Stickstoffdruck von 10 bar auf 80°C erhitzt und 5 Stunden auf dieser Temperatur gehalten, wobei sich ein Druck von 18 bar einstellte. Beim Entspannen bei 80°C durch das Bodenventil wurde das $\beta$-Hydroxy-$C_{11-14}$-alkyl-trimethylammoniumchlorid als hygroskopisches weißes Pulver erhalten. Das Produkt hatte die Aminzahl 1,8 und löste sich klar in Wasser.

### Beispiel 7

Als Ausgangsmaterial diente ein Dimethyl-$\beta$-hydroxy-$C_{15-18}$-alkylamin (Aminzahl 179), das aus einem Gemisch von $C_{15-18}$-Epoxyalkanen mit nichtendständigen Epoxygruppierungen in willkürlicher Verteilung durch Reaktion mit Dimethylamin erhalten worden war. 470 g (1,5 Mol) dieses tertiären Amins und 455 g (9 Mol) Methylchlorid wurden wie in Beispiel 5 unter einem Stickstoffdruck von 10 bar auf 80°C erhitzt und 5 Stunden auf dieser Temperatur gehalten, wobei sich ein Druck von 21 bar einstellte. Durch Entspannen bei 80°C über das Bodenventil wurde das $\beta$-Hydroxy-$C_{15-18}$-alkyl-trimethylammoniumchlorid als gelbliches Pulver mit der Aminzahl 18 erhalten.

### Patentansprüche

1. Verfahren zur Herstellung von quaternären Ammoniumhalogeniden in Pulver- oder Granulatform durch Umsetzung von tertiären Aminen mit einem Überschuß an Alkylhalogeniden in Abwesenheit von Lösungsmitteln bei erhöhter Temperatur und erhöhtem Druck, dadurch gekennzeichnet, daß man tertiäre Amine, die neben zwei Alkyl- oder Hydroxyalkylresten mit 1 bis 3 Kohlenstoffatomen einen Alkylrest oder $\beta$-Hydroxyalkylrest mit 8 bis 18 Kohlenstoffatomen enthalten, mit Methylchlorid, Methylbromid, Methyljodid, Ethylchlorid oder Ethylbromid zur Reaktion bringt und anschließend das unter Überdruck stehende heiße Reaktionsgemisch in einen unter geringerem Druck stehenden Behälter ausströmen läßt.

2. Verfahren nach Anspruch 1, dadurch gekennzeichnet, daß man das Reaktionsgemisch in einen unter Normaldruck stehenden Behälter ausströmen läßt.

### Claims

1. A process for the production fo quaternary ammonium halides in powder or granulate form by reacting tertiary amines with an excess of alkyl halides in the absence of solvents at elevated temperature and pressure, characterised in that tertiary amines containing an alkyl radical or $\beta$-hydroxyalkyl radical with 8 to 18 carbon atoms in addition to two alkyl or hydroxyalkyl radicals with 1 to 3 carbon atoms are reacted with methyl chloride, methyl bromide, methyl iodide, ethyl chloride or ethyl bromide, after which the hot reaction mixture under excess pressure is allowed to flow out into a vessel under lower pressure.

2. A process as claimed in Claim 1, characterised in that the reaction mixture is allowed to flow out into a vessel under normal pressure.

### Revendications

1. Procédé de préparation d'halogénures d'ammonium quaternaires sous forme de poudre ou de granules par réaction d'amines tertiaires avec un excès d'halogénure d'alkyle en l'absence de solvant à température élevée et pression élevée, caractérisé en ce que l'on fait réagir des amines tertiaires contenant, outre deux groupes alkyle ou hydroxyalkyle en $C_1-C_3$, un groupe alkyle ou $\beta$-hydroxyalkyle en $C_8-C_{18}$, avec le chlorure de méthyle, le bromure de méthyle, l'iodure de méthyle, le chlorure d'éthyle ou le bromure d'éthyle et on laisse ensuite écouler le mélange de réaction chaud et sous pression dans un récipient placé sous une pression plus basse.

2. Procédé selon la revendication 1, caractérisé en ce qu'on laisse écouler de mélange de réaction dans un récipient à pression normale.